# EUROPEAN PATENT APPLICATION

(11) **EP 4 840 879 A2**
(43) Date of publication of application: **18.11.2026**
(21) Application number: 26208516.0
(22) Date of filing: 25.08.2022
(51) Int. Cl.: C07K 14/47

(54) **VACCINE AGAINST PANCREATIC CANCER, AND MEDICAL USE THEREOF**

(30) Priority: 04.11.2021 CN 202111301004
(62) Document number(s) of the earlier application(s) in accordance with Art. 76 EPC: 22888956.4 / 4 427 761
(71) Applicant: Yuanben (Zhuhai Hengqin) Biotechnology Co., Ltd., Zhuhai, Guangdong 519000 (CN)
(72) Inventor: CAI, Jiong, Taizhou, Jiangsu 225300 (CN)
(74) Representative: Gille Hrabal Partnerschaftsgesellschaft mbB Patentanwälte

(57) **Abstract**

An anti-tumor fusion protein, wherein the fusion protein can inhibit the growth of MUC1-positive tumor cells, and can inhibit the growth of pancreatic cancer tumor cells. The fusion protein has broad application prospects for the prevention and/or treatment of pancreatic cancer.

## Description

The present application claims priority to the prior application with the patent application No. 202111301004X entitled "VACCINE AGAINST PANCREATIC CANCER, AND MEDICAL USE THEREOF" filed to China National Intellectual Property Administration on November 4, 2021, which is incorporated herein by reference in its entirety.

### TECHNICAL FIELD

The present disclosure relates to use of a fusion protein, in particular to use of a fusion protein comprising human MUC1 for the manufacture of a medicament for preventing and treating pancreatic cancer.

### BACKGROUND

Five-year survival rate of pancreatic cancer is less than 5%, and it is known as "the king of carcinoma". Early symptoms of pancreatic cancer are not significant and are easily ignored, and pancreatic cancer is usually diagnosed at an advanced stage of cancer. The periphery of pancreas is rich in blood vessels, nerves, and important organs such as kidney, liver, and the like, so that the surgery is difficult, and only 10%-15% of patients at the early stage are suitable for surgical treatment because of the late discovery generally. Most patients can only be treated with gemcitabine, which is combined with carboplatin, capecitabine, and the like, but in either case, the therapeutic effect is only slightly better than no treatment. Survival time for patients with advanced and metastatic pancreatic cancer is generally only a few months. Pancreatic cancer, as it progresses, utilizes immune, vascular, and connective tissue post-injury repair responses of the surrounding tissue matrix to create a favorable tumor microenvironment to facilitate tumor growth. Although immune checkpoint therapy has a good therapeutic effect in a variety of cancers, it has a very poor therapeutic response to pancreatic cancer.

Tumor cell vaccines are likely to activate the immune system, making them directly accessible to cancer cells. The tumor vaccines induce tumor-specific immune responses, and activate the immune system to attack cancer cells with special antigens. Among the numerous immunizing antigens, mucin MUC1 is given hope. MUC1 was originally found to have functions of protecting and lubricating the epithelium. It was subsequently found to play an important role in cell signaling and in all stages of tumorigenesis from malignant cells transformed to tumor spreading. MUC1 is a highly glycosylated transmembrane protein, a type I transmembrane protein with a highly glycosylated extracellular domain that extends 200-500 nm from the cell surface. The full length is divided into an extracellular region, a transmembrane region, and an intracellular region. The extracellular region is composed of the proline, threonine, and serine-rich (PTS) domain and the SEA domain. The PTS domain, also known as variable number tandem repeat (VNTR) region, is encoded by a highly polymorphic exon that encodes multiple 20-21 amino acid sequence repeats, and the intracellular region (CT) of MUC1 is highly conserved. Overexpression of MUC1 is commonly associated with colon cancer, breast cancer, ovarian cancer, lung cancer, and pancreatic cancer. MUC1 is proved to be present in various adenocarcinomas. In a study of treating patients with primary liver cancer, the proportion of patients with high expression of MUC1 was as high as 68%. Meanwhile, the recurrence rate after surgery was the highest, which was positively correlated with the expression intensity of MUC1. In addition, MUC1 is also overexpressed in some hematological malignancies. There are not only differences in the expression levels of MUC1 between tumor tissues and normal tissues, but also differences in the glycosylation of MUC1 between tumor tissues and normal tissues. The low-glycosylated MUC1 is overexpressed on the whole surface of tumor cells, so that the adhesion of the tumor cells is reduced, thereby providing convenience for tumor metastasis. In the formed tumors, a large amount of MUC1 can inhibit the killing effect of NK cells on tumor cells, can also inhibit the proliferation of cytotoxic lymphocytes (CTLs), and can even induce the apoptosis of CTLs.

Vaccination can provide a long-term protection effect to the host with few side effects, and is one of the important methods for treating cancers. In theory, MUC1 should be a very good vaccine antigen, but the clinical effect in human clinical trials is not ideal. Most antibodies generated by the vaccine cannot well bind to tumor cells, and cannot effectively protect the body to kill tumors.

Our studies indicate that MUC1 can be linked to proteins on bacteria or viruses to generate T cell responses against cancer cells, producing anti-tumor effects. Neoantigen vaccine, which is currently under intense research, requires high-throughput sequencing and bioinformatics screening, which requires a lot of time and resources, making it difficult to benefit patients. Therapeutic cancer vaccines with lower development costs and better efficacy are expected. Disclosed in the present disclosure is a gene optimization method for a recombinant protein that can be used for preparing a low-cost therapeutic cancer vaccine, be suitable for producing by Escherichia coli, and inhibit the growth of pancreatic cancer cells.

### SUMMARY

In order to provide a more effective biological formulation for preventing and treating pancreatic cancer, the present disclosure provides use of a fusion protein in the manufacture of an anti-tumor medicament, particularly a pancreatic cancer medicament. The present disclosure is implemented by the following technical solutions:
the use of a fusion protein in the manufacture of a medicament for preventing and/or treating a tumor.

According to the present disclosure, the tumor is a MUC1-positive tumor, such as an adenocarcinoma expressing MUC1 or a hematological tumor expressing MUC1; more preferably, the tumor is pancreatic cancer.

The present disclosure further provides use of a fusion protein in the manufacture of a medicament for preventing and/or treating pancreatic cancer.

According to the present disclosure, the fusion protein comprises maltose-binding protein MBP and/or protein MUC1-N.

According to the present disclosure, the fusion protein is formed by connecting the maltose-binding protein MBP and/or the protein MUC1-N in tandem.

Furthermore, the nucleotide sequence of the MUC1-N gene is set forth in SEQ ID NO.1, and the nucleotide sequence of the MBP gene is set forth in SEQ ID NO.2.

Furthermore, the amino acid sequence of the fusion protein is set forth in SEQ ID NO.3.

The present disclosure further provides a method for inhibiting the growth of tumor cells of pancreatic cancer, which comprises administering the fusion protein described herein.

The present disclosure provides a pharmaceutical composition comprising the fusion protein of the present disclosure.

According to the present disclosure, the medicament is used for treating a tumor; preferably, the tumor is a MUC1-positive tumor, such as an adenocarcinoma expressing MUC1 or a hematological tumor expressing MUC1, more preferably pancreatic cancer.

According to the present disclosure, the medicament is used for treating breast cancer.

According to the present disclosure, the fusion protein comprises maltose-binding protein MBP and/or protein MUC1-N.

According to the present disclosure, the fusion protein is formed by connecting the maltose-binding protein MBP and/or the mucin MUC1-N in tandem.

Furthermore, the nucleotide sequence of the MUC1-N gene is set forth in SEQ ID NO.1, and the nucleotide sequence of the MBP gene is set forth in SEQ ID NO.2.

More preferably, the amino acid sequence of the fusion protein is set forth in SEQ ID NO.3.

The present disclosure further provides use of a fusion protein in a healthcare product, a cosmetic product, food, or a food additive, characterized in that wherein the fusion protein comprises maltose-binding protein MBP and/or protein MUC1-N.

According to the present disclosure, the healthcare product, the cosmetic product, the food, or the food additive can be used for inhibiting a tumor.

Preferably, the tumor is a MUC1-positive tumor, such as an adenocarcinoma expressing MUC1 or a hematological tumor expressing MUC1, more preferably pancreatic cancer.

In a preferred embodiment of the present application, the fusion protein is formed by connecting the maltose-binding protein MBP and the protein MUC1-N in tandem.

Furthermore, the nucleotide sequence of the MUC1-N gene is set forth in SEQ ID NO.1, and the nucleotide sequence of the MBP gene is set forth in SEQ ID NO.2.

More preferably, the amino acid sequence of the fusion protein is set forth in SEQ ID NO.3.

The present disclosure further provides use of protein MBP gene and/or protein MUC1-N gene in the manufacture of a medicament for preventing and/or treating a tumor, characterized in that the nucleotide sequence of the MUC1-N gene is set forth in SEQ ID NO.1, and the MBP gene is set forth in SEQ ID NO.2.

According to the present disclosure, the cancer comprises all cancers expressing MUC1, including adenocarcinomas expressing MUC1 or hematological tumors expressing MUC1, more preferably pancreatic cancer.

The present disclosure further provides use of protein MBP gene and/or protein MUC1-N gene in the manufacture of a medicament for preventing and/or treating pancreatic cancer, characterized in that the nucleotide sequence of the MUC1-N gene is set forth in SEQ ID NO.1, and the nucleotide sequence of the MBP gene is set forth in SEQ ID NO.2.

### Beneficial Effects of Present Disclosure:

The present disclosure proves, through animal experiments, that the fusion protein and genes of the present disclosure have significant inhibitory effects on the growth of pancreatic cancer cells with different doses, and have significant preventive and therapeutic effects on pancreatic cancer.

### BRIEF DESCRIPTION OF THE DRAWINGS

FIG. 1 shows gel diagrams after protein expression and purification;
FIG. 2 shows the experiment of inhibiting the proliferation of pancreatic cancer cells by MBP-Muc1-N;
FIG. 3 shows the experiment of inhibiting the proliferation of pancreatic cancer cells by MBP-Muc1-N;
FIG. 4 shows the experimental results of single-drug therapy for pan-02 pancreatic cancer by dual adjuvant vaccines.

### DETAILED DESCRIPTION

The present disclosure is illustrated by the following examples. However, it is known to those skilled in the art that the following examples are not intended to limit the scope of the present disclosure, and any modifications and variations made on the basis of the examples of the present disclosure are within the scope of the present disclosure.

### Example 1. Construction and Expression of Fusion Protein

### 1. Gene optimization

The nucleotide sequence of the optimized MUC1-N protein is set forth in SEQ ID NO.1:

ggtgttacttctgctcctgatactcgtcctgctcctggttctactgcaccgccagcacatggcgtgacgtctgcgccagatacccgtccggcac
cgggttccaccgccccaccggcacacggcgtaacctccgcgccagacacccgtccagcgccaggttctaccgctccgcctgctcatggtg
ttacctctgccccggacactcgtccggctccaggttctactgccccgccagctcatggcgtcacttccgccccggatacccgtcctgccccg
ggctctactgcgcctccggctcacggcgttacctctgcaccggatactcgtccggctccgggctctaccgcaccacctgctcatggcgtaac
gagcgctcctgatacccgtccggctccgggttccactgcacctccggcccac

The nucleotide sequence of the optimized MBP protein is set forth in SEQ ID NO.2:

aaaatcgaagaaggcaaactggtgatctggatcaacggtgataagggttataacggtctggcggaagtaggcaagaaattcgaaaaagaca
ccggtatcaaagttaccgttgaacatccagacaaactggaagaaaaattccctcaggtggcggctaccggcgacggccctgatatcattttct
gggcacatgatcgttttggcggttacgcgcagtctggcctgctggcagaaatcacgccggataaggcgttccaggacaaactgtaccctttta
cctgggacgcggtgcgttacaacggcaaactgatcgcttacccgatcgcagtggaagctctgtccctgatctacaataaggacctgctgccg
aacccgcctaaaacgtgggaagaaatcccggccctggacaaagaactgaaagcaaaaggtaagagcgctctgatgttcaatctgcaggaa
ccgtacttcacttggccgctgatcgcagctgacggcggttatgcgtttaaatacgaaaacggtaaatatgacattaaggacgtcggcgttgata
acgccggcgccaaagcgggcctgacctttctggtcgacctgatcaaaaacaaacacatgaacgctgacaccgattattctattgcggaggc
ggcttttaacaagggcgagaccgcaatgaccatcaacggtccgtgggcttggtctaacatcgacacctccaaagtaaattacggtgttaccgt
cctgccgaccttcaaaggtcaaccgagcaaaccgttcgtgggcgtgctgtccgcaggtatcaacgctgcctccccaaacaaagagctggcc
aaagagttcctggaaaactatctgctgaccgacgaaggcctggaagctgttaataaagacaaaccgctgggtgctgttgcactgaaatcctat
gaagaagaactggtcaaagatccgcgtattgccgccactatggagaacgcgcagaaaggtgaaatcatgccgaacatcccgcaaatgtcc
gctttttggtacgcggtgcgtaccgctgtaattaacgcggcgtccggtcgtcagactgtcgatgaagcgctgaaagatgctcagactaactcta
gctctaacaataacaataataacaacaacaacaatctgggtattgaaggtcgcatctct

### Synthesis of optimized gene sequence of MUC1-N fused to MBP

To achieve sequential tandem expression of MBP and Muc1-N, the sequence of the obtained fusion protein is set forth in SEQ ID NO.3:

KIEEGKLVIWINGDKGYNGLAEVGKKFEKDTGIKVTVEHPDKLEEKFPQVAATGDGPDIIF
WAHDRFGGYAQSGLLAEITPDKAFQDKLYPFTWDAVRYNGKLIAYPIAVEALSLIYNKDL
LPNPPKTWEEIPALDKELKAKGKSALMFNLQEPYFTWPLIAADGGYAFKYENGKYDIKDV
GVDNAGAKAGLTFLVDLIKNKHMNADTDYSIAEAAFNKGETAMTINGPWAWSNIDTSKV
NYGVTVLPTFKGQPSKPFVGVLSAGINAASPNKELAKEFLENYLLTDEGLEAVNKDKPLGA
VALKSYEEELVKDPRIAATMENAQKGEIMPNIPQMSAFWYAVRTAVINAASGRQTVDEAL
KDAQTNSSSNNNNNNNNNNLGIEGRISGVTSAPDTRPAPGSTAPPAHGVTSAPDTRPAPGS

TAPPAHGVTSAPDTRPAPGSTAPPAHGVTSAPDTRPAPGSTAPPAHGVTSAPDTRPAPGSTA
PPAHGVTSAPDTRPAPGSTAPPAHGVTSAPDTRPAPGSTAPPAH.

Tandem synthesis was performed based on the gene sequence of the MBP and Muc1-N fusion protein. For this purpose, oligonucleotide sequences 1a_1, 1a_2, 1a_3, 1a_4, 1a_5, 1a_6, 1a_7, 1a_8, 1a_9, 1a_10, 1a_11, 1a_12, 1a_13, 1a_14, 1a_15, 1a_16, 1a_17, 1a_18, 1a_19, 1a_20, 1a_21, 1a_22, 1a_23, 1a_24, 1a_25, 1a_26, 1a_27, 1a_28, 1a_29, and 1a_30 were synthesized first, then sequences 1b_1, 1b_2, 1b_3, and 1b_4 were synthesized, and gene amplification was performed using 1-seq2 and 1-R sequences to obtain the optimized gene sequence of MUC1-N fused to MBP.

| .No. | Sequence | Number of bases |
|---|---|---|
| 1-seq2 | tacttcacttggccgctgat | 20 |
| 1-R | ggcctctgcagtcgacgggcccggggaagacttggacgcgtatccggtgcagaagtaacgccgtgtgcc ggcggtgcagtggaacccggcgctggacgagtatccggcgcagacgt | 116 |
| 1a_1 | aacgacggccagagaattcgagctcggtaccggatccctcctcgctgcccagccggcgatggcc | 64 |
| 1a_2 | cccttatcaccgttgatccagatcaccagtttgccttcttcgattttatccatggccatcgccggctg | 68 |
| 1a_3 | caacggtgataagggttataacggtctggcggaagtaggcaagaaattcgaaaaagacaccggtatca | 68 |
| 1a_4 | acctgagggaatttttcttccagtttgtctggatgttcaacggtaactttgataccggtgtctt | 64 |
| 1a_5 | aaaattccctcaggtggcggctaccggcgacggccctgatatcattttctgggcacatgatcgttttg | 68 |
| 1a_6 | aacgccttatccggcgtgatttctgccagcaggccagactgcgcgtaaccgccaaaacgatcatgtg | 67 |
| 1a_7 | gccggataaggcgttccaggacaaactgtacccttttacctgggacgcggtgcgttacaacggcaa | 66 |
| 1a_8 | cttattgtagatcagggacagagcttccactgcgatcgggtaagcgatcagtttgccgttgtaacgc | 67 |
| 1a_9 | ctgatctacaataaggacctgctgccgaacccgcctaaaacgtgggaagaaatcccggccctggacaaa | 69 |
| 1a_10 | cggttcctgcagattgaacatcagagcgctcttaccttttgctttcagttctttgtccagggccgg | 66 |
| 1a_11 | aatctgcaggaaccgtacttcacttggccgctgatcgcagctgacggcggttatgcgtttaaatacg | 67 |
| 1a_12 | tttggcgccggcgttatcaacgccgacgtccttaatgtcatatttaccgttttcgtatttaaacgcat | 68 |
| 1a_13 | aacgccggcgccaaagcgggcctgacctttctggtcgacctgatcaaaaacaaacacatgaacgct | 66 |
| 1a_14 | gcggtctcgcccttgttaaaagccgcctccgcaatagaataatcggtgtcagcgttcatgtgttt | 65 |
| 1a_15 | caagggcgagaccgcaatgaccatcaacggtccgtgggcttggtctaacatcgacacctccaa | 63 |
| 1a_16 | ttgctcggttgacctttgaaggtcggcaggacggtaacaccgtaatttactttggaggtgtcgatg | 66 |
| 1a_17 | aggtcaaccgagcaaaccgttcgtgggcgtgctgtccgcaggtatcaacgctgcctccccaaacaaa | 67 |
| 1a_18 | ccttcgtcggtcagcagatagttttccaggaactctttggccagctctttgtttggggaggc | 62 |
| 1a_19 | gctgaccgacgaaggcctggaagctgttaataaagacaaaccgctgggtgctgttgcactgaaa | 64 |
| 1a_20 | ctccatagtggcggcaatacgcggatctttgaccagttcttcttcataggatttcagtgcaacagc | 66 |
| 1a_21 | gccgccactatggagaacgcgcagaaaggtgaaatcatgccgaacatcccgcaaatgtccgc | 62 |
| 1a_22 | ctgacgaccggacgccgcgttaattacagcggtacgcaccgcgtaccaaaaagcggacatttgcggg | 67 |
| 1a_23 | gcgtccggtcgtcagactgtcgatgaagcgctgaaagatgctcagactaactctagctctaaca | 64 |
| 1a_24 | gagatgcgaccttcaatacccagattgttgttgttgttattattgttattgttagagctagagt | 64 |
| 1a_25 | tgaaggtcgcatctctggcgttactagcgcaccggatacccgtccggcaccgggctctaccgct | 64 |
| 1a_26 | cggagcggaagtaacaccgtgagcaggcggagcggtagagcccgg | 45 |
| 1a_27 | tgttacttccgctccggacacccgtccagcgccaggttccaccgcaccgccggcacacggcgttacctcc gctccagatactcgcc | 86 |
| 1a_28 | agtatccggcgcagacgtgacgccgtgcgctggcggtgcagtagaacccggtgccgggcgagtatctgg ag | 71 |
| 1a_29 | tctgcgccggatactcgtccagcgccgggttccactgcaccgccggcacacggcgttacttctgcaccgg atacgcgtccaagtcttcc | 89 |
| 1a_30 | ggcctctgcagtcgacgggcccggggaagacttggacgc | 39 |
| 1b_1 | ccaatggtctcagtccggctccaggttctactgccccgccggcacatggcgttacctctg | 60 |
| 1b_2 | cgtgcgccggaggagcggtgctgcctggtgcagggcgagtgtccggggcagaggtaacgccat | 63 |
| 1b_3 | ctcctccggcgcacggtgtcacttctgctccagacaccc | 39 |
| 1b_4 | ggccgcaagcttgtcgacggagctcgaattctcagtgcgccggcggggcggtgctgcccggagccgga cgggtgtctggagcag | 84 |

### 2. Recombinant expression and purification of proteins

A NcoI restriction site was added to a 5' PCR primer of the fusion gene, and an EcoI restriction site was added to a 3' PCR primer. The amplified gene was subjected to double digestion, and then inserted into a pET26b(+) Escherichia coli expression vector subjected to the same double digestion. The bacteria were screened in resistant bacterial culture plates and subjected to monoclonal selection. The bacteria were cultured using a kanamycin-resistant medium, and the expression of the operon was induced by IPTG. The non-optimized sequence and the optimized sequence were subjected to experiments. The obtained final whole bacterial liquid was pretreated with a buffer containing SDS at 95 °C and analyzed by 5%-12% polyacrylamide gel electrophoresis. The results show that: the expression level of the MBP-Muc1-N sequence before optimization was only 2% of the total protein, and the expression level of the sequence 1 MBP-Muc1-N after optimization accounted for 51% of the total protein, which was increased by 25.5 times. After purification by an affinity column, the protein expressed by the non-optimized gene must be subjected to 10-fold concentration before loading for observation, the yield of the purified protein after concentration was 0.8 mg of 100 mL culture, and the yield of the optimized MBP-Muc1-N sequence was 9.6 mg, which was increased by 12 times.

### Example 2. Activity Assay of MBP-Muc1-N Fusion Protein

### 1. Materials

Experimental reagents: the recombinant MBP-MUC1-N fusion protein prepared by using the method of the present application; Pan-02 pancreatic cancer cells were purchased from National Experimental Cell Resource Center; the normal saline for injection was purchased from Beijing Tiantan Biological Products Co., Ltd.

Experimental animals: C57 BL/6J mice were purchased from Beijing Huafukang Biotechnology Co., Ltd.

### 2. Methods

(1) Each group consisted of 6 male C57BL/6J mice aged 6-8 weeks. The number of Pan-02 pancreatic cancer cells inoculated was 1.75 × 10⁵ cells/mouse, the inoculation site was subcutaneous at the right axilla, and MBP-Muc1-N was inoculated at 50 µg per dose. On day 7 after inoculation, it can be seen that the tumor was about 5 mm, and a first dose of MBP-Muc1-N was injected intramuscularly to the leg; on day 50 after inoculation, a second dose of MBP-Muc1-N was injected; thereafter, third and fourth doses of MBP-Muc1-N were injected on day 54 and day 57 after inoculation, respectively.
(2) Each group consisted of 6 male C57BL/6J mice aged 6-8 weeks. The number of Pan-02 pancreatic cancer cells inoculated was 1.0 × 10⁶ cells/mouse, the inoculation site was subcutaneous at the right axilla, and MBP-Muc1-N was inoculated at 50 µg per dose. On day 7 after inoculation, it can be seen that the tumor was about 5 mm; on day 17 after inoculation, a second dose of MBP-Muc1-N was injected; thereafter, third, fourth, fifth, and sixth doses of MBP-Muc1-N were injected on day 20, day 24, day 27, and day 31 after inoculation, respectively.

### 3. Assay results

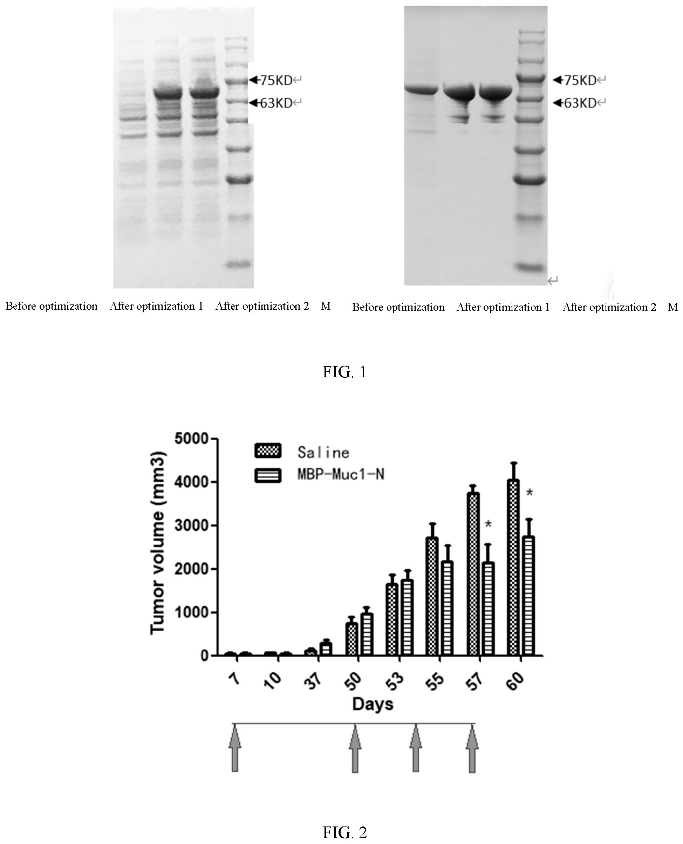

3.1 In method 1, as can be seen from FIG. 2, on day 7 after the mice were inoculated with the Pan-02 pancreatic cancer cells at 1.75 × 10⁵ cells/mouse, it can be seen that the tumor was about 5 mm, and the first dose of MBP-Muc1-N was injected intramuscularly to the leg; thereafter, it can be seen that the tumor grew slowly until day 50 after inoculation, then the tumor grew rapidly, and the second dose of MBP-Muc1-N was injected; thereafter, the third and fourth doses of MBP-Muc1-N were injected on day 54 and day 57 after inoculation, respectively. After the injection of the third and fourth doses of MBP-Muc1-N, it can be seen that the tumor growth was significantly inhibited.

**Table 1. Effect of long-term administration on pancreatic cancer**

| | Control group | MBP-Muc1-N group | Inhibition rate (%) |
|---|---|---|---|
| Day 57 | 3739±460 mm³ | 2131±1087mm³ | 43% |
| Day 60 | 4075±983 mm³ | 2739±970mm³ | 33% |

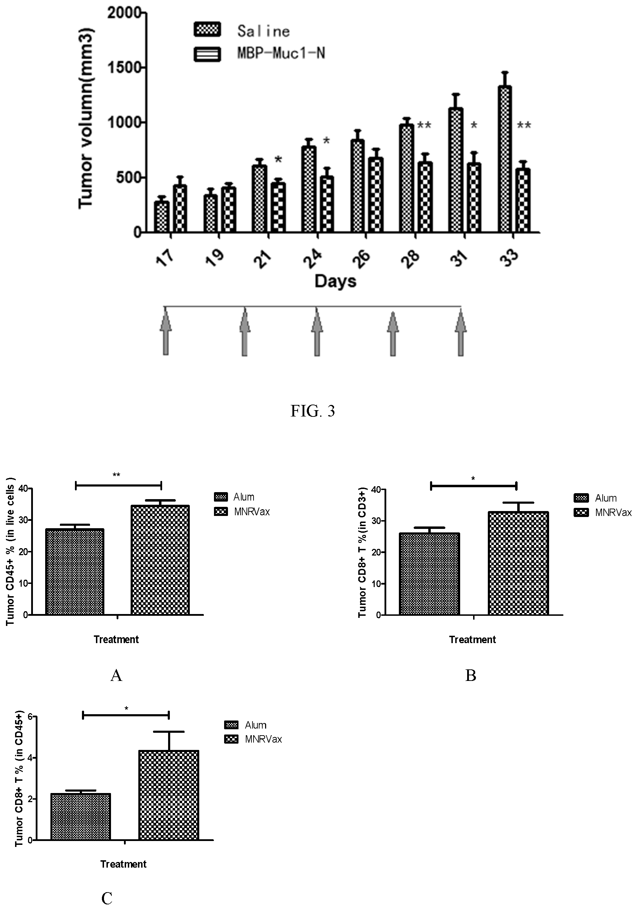

3.2 In method 2, as can be seen from FIG. 3, on day 7 after inoculation, it can be seen that the tumor was about 5 mm; thereafter, the tumor grew slowly until day 17 after inoculation, and the second dose of MBP-Muc1-N was injected; thereafter, the third, fourth, fifth, and sixth doses of MBP-Muc1-N were injected on day 20, day 24, day 27, and day 31 after inoculation, respectively. After the injection of the second, third, fourth, fifth, and sixth doses of MBP-Muc1-N, it can be seen that the tumor growth was significantly inhibited.

**Table 2. Effect of short-term administration on pancreatic cancer**

| | Control group | MBP-Muc1-N group | Inhibition rate (%) |
|---|---|---|---|
| Day 21 | 610±141 mm³ | 450±90mm³ | 26% |
| Day 24 | 780±159 mm³ | 508±191mm³ | 35% |
| Day 28 | 980±149mm³ | 632±205mm³ | 36% |
| Day 31 | 1127±318mm³ | 625±249mm³ | 45% |
| Day 33 | 1330±318mm³ | 577±172mm³ | 57% |

In conclusion, the present disclosure determines, through the animal experiments of the modified MBP-Muc1-N, that the modified fusion protein has a significant inhibitory effect on the growth of pancreatic cancer cells with different concentrations, and can effectively prevent and/or treat pancreatic cancer.

### Example 3. Single-Drug Therapy for pan-02 Pancreatic Cancer by Single Adjuvant Vaccines 1. Materials

Experimental reagents: a recombinant MBP-MUC1-N fusion protein expression strain prepared by using the method of the present application; an anti-PD-1 antibody was purchased from Bioxcell; pan-02 pancreatic cancer cells were purchased from National Experimental Cell Resource Center; an aluminum hydroxide adjuvant was purchased from Corda.

Experimental animals: C57BL/6J mice were purchased from Beijing Huafukang Biotechnology Co., Ltd.

### 2. Methods

### 2.1 Production of MBP-MUC1-N fusion proteins

The recombinant MBP-MUC1-N fusion protein expression strain prepared using the method of the present application was subjected to fermentation, and a fermentation broth was collected and subjected to bacterial cell clarification and disruption. The supernatant was collected by centrifugation and loaded onto amylose-resin. The affinity purification of the MBP-MUC1-N fusion protein was completed by maltose elution. Subsequently, the buffer was exchanged by dialysis. The sample was loaded onto Q-sepharose, and anion exchange purification was completed with high-concentration NaCl. Then the buffer was exchanged to a citric acid buffer. The sample was loaded onto SP-sepharose, and cation exchange purification was completed with high-concentration arginine. Endotoxin, residual host DNA, and residual proteins in the purified product all meet the requirements of the stock solution. The buffer was exchanged to a 20 mM acetic acid-sodium acetate buffer with 150 mM arginine by dialysis.

### 2.2 Immunization of mice

Mice were weighed and randomly divided into groups of 10. pan-02 pancreatic cancer cells were diluted with PBS and inoculated in the right axilla of the C57BL/J mice according to 1 × 10⁶ cells/mouse, with a total amount of 100 µL/mouse, for modeling. After the tumor diameter reached 5 mm (10 days), the mice were divided into 5 groups, namely an aluminum hydroxide control group (100 µL, twice weekly, intramuscular injection), an MNRvax low-dose group (0.2 mg/kg, 100 µL, twice weekly, intramuscular injection), an MNRvax medium-dose group (2 mg/kg, 100 µL, twice weekly, intramuscular injection), an MNRvax high-dose group (8 mg/kg, 100 µL, twice weekly, subcutaneous injection), and an immune checkpoint inhibitor anti-PD-1 antibody group (10 mg/kg, 100 µL, twice weekly, intraperitoneal injection).

The medicaments were injected on day 10, day 13, day 17, and day 20 of tumor bearing, and the tumor size was measured on day 10, day 13, day 17, day 20, and day 24. The tumor graft inhibition rate and the tumor inhibition rate of the subcutaneous pancreatic cancer in each group were calculated. The formula is as follows: [tumor inhibition rate = (mean tumor weight in control group - mean tumor weight in experimental group)/mean tumor weight in control group × 100%]. [tumor cure rate = (number of mice without tumor/10 mice) × 100%].

### 3. Results

**Table 3. Tumor weights (g), tumor inhibition rates (%) and tumor cure rates in mice**

| | Tumor volume (cm³) | Tumor inhibition rate | Tumor cure rate |
|---|---|---|---|
| Adjuvant group | 0.408±0.091 | 0 | 0 |
| Low-dose vaccine group | 0.332±0.068 | 17 | 20 |
| Medium-dose vaccine group | 0.327±0.091 | 20 | 20 |
| High-dose vaccine group | 0.320±0.112 | 22 | 20 |
| PD-1 group | 0.338±0.087 | 17 | 10 |

**Table 4. Therapeutic effects at different doses**

| cm³ | Control group | Low-dose | Medium-dose | High-dose | PD-1 antibody |
|---|---|---|---|---|---|
| | | vaccine group | vaccine group | vaccine group | group |
| Day 10 | 0.172±0.014 | 0.172±0.014 | 0.172±0.014 | 0.172±0.016 | 0.172±0.014 |
| Day 13 | 0.177±0.015 | 0.179±0.019 | 0.180±0.013 | 0.180±0.021 | 0.180±0.033 |
| Day 17 | 0.213±0.015 | 0.213±0.034 | 0.217±0.038 | 0.234±0.073 | 0.204±0.049 |
| Day 20 | 0.359±0.079 | 0.295±0.072 | 0.286±0.062* | 0.291±0.083 | 0.301±0.075 |
| Day 24 | 0.408±0.091 | 0.332±0.068* | 0.327±0.091 | 0.320±0.112 | 0.338±0.087 |

| | | | | | |
|---|---|---|---|---|---|
| * p < 0.05 (vs control group) | | | | | |

As can be seen from Table 3 and Table 4, the low-dose, medium-dose, and high-dose MBP-Muc1-N vaccine treatment groups could significantly eliminate tumors compared to the control group. The tumors were significantly reduced on day 20 and day 24 of tumor bearing. The tumors were reduced from 0.359±0.079 mm³ to 0.286±0.062 mm³, 0.295±0.072 mm³, and 0.291±0.083 mm³; and from 0.408±0.091 mm³ to 0.332±68 mm³, 0.327±0.091 mm³, and 0.320±0.112 mm³, respectively. The p values were 0.077, 0.036, and 0.081, as well as 0.049, 0.062, and 0.069, respectively, with significant differences. The tumors in the PD-1 antibody treatment group were reduced from 0.359±0.079 mm³ to 0.301±0.075 mm³, and from 0.408±0.091 mm³ to 0.338±0.087 mm³, indicating that the MBP-Muc1-N vaccine is more effective than the PD-1 antibody.

### Example 4. Single-Drug Therapy for pan-02 Pancreatic Cancer by Dual Adjuvant Vaccines

### 1. Materials

Experimental reagents: the preparation method for the recombinant MBP-MUC1-N fusion protein was the same as above; an aluminum hydroxide adjuvant was purchased from Corda; a CpG1826 adjuvant (5'-TCCATGACGTTCCTGACGTT-3') and a thio-*CpG1826* adjuvant (5'-T*C*C*A*T*G*A*C*G*T*T*C*C*T*G*A*C*G*T*T-3') were purchased from Shanghai Sangon Biotech.

Experimental animals: C57BL/6J mice were purchased from Beijing Huafukang Biotechnology Co., Ltd.

### 2. Methods

### 2.1 Production of MBP-MUC1-N fusion proteins

The recombinant MBP-MUC1-N fusion protein was obtained by a three-step purification method. Endotoxin, residual host DNA, and residual proteins in the purified product all meet the requirements of the stock solution.

### 2.2 Immunization of mice

Mice were weighed and randomly divided into groups of 6. pan-02 pancreatic cancer cells were diluted with PBS and inoculated in the right axilla of the C57BL/J mice according to 1 × 10⁶ cells/mouse, with a total amount of 100 µL/mouse, for modeling. After the tumor diameter reached 5 mm (10 days), the mice were divided into 6 groups, namely a PBS control group (100 µL, twice weekly, intramuscular injection), an MNRvax group (2 mg/kg, 100 µL, twice weekly, intramuscular injection), a CpG group (10 µg/mouse, 100 µL, twice weekly, intramuscular injection), a *CpG* group (10 µg/mouse, 100 µL, twice weekly, intramuscular injection), an MNRvax + CpG group, and an MNRvax + *CpG* group.

The medicaments were injected on day 8, day 21, day 29, and day 36 of tumor bearing, and the tumor size was measured on day 43. The tumor graft inhibition rate and the tumor inhibition rate of the subcutaneous pancreatic cancer in each group were calculated. The formula is as follows: [tumor inhibition rate = (mean tumor weight in control group - mean tumor weight in experimental group)/mean tumor weight in control group × 100%]. [tumor cure rate = (number of mice without tumor/10 mice) × 100%].

### 3. Results

**Table 5. Tumor weights (g), tumor inhibition rates (%) and tumor cure rates in mice**

| | Tumor volume (cm³) | Tumor inhibition rate | Tumor cure rate |
|---|---|---|---|
| PBS group | 2.26±0.78 | 0 | 0 |
| MNRVax vaccine group | 2.06±0.34 | 9 | 0 |
| CpG group | 1.50±0.58 | 34 | 17 |
| *CpG* group | 0.99±0.79*# | 56 | 33 |
| MNRVax + CpG group | 0.75±0.39**###¶ | 67 | 33 |
| MNRVax + *CpG* group | 1.21±0.43*## | 46 | 0 |

| | | | |
|---|---|---|---|
| *p< 0.05; **p < 0.01 (vs PBS) #p < 0.05; ##p < 0.01; ###p < 0.001 (vs MNRVax). ¶p < 0.05 (vs CpG) | | | |

### 4. Conclusion

The recombinant MBP-MUC1-N fusion protein vaccine prepared by an acetic acid-sodium acetate buffer system has a low inhibitory effect on tumors, and the inhibitory effect can be improved by adding CpG1826.

### Example 5. Study on Mechanism of Single-Drug Therapy for pan-02 Pancreatic Cancer by Vaccines

### 1. Materials

Experimental reagents: the recombinant MBP-MUC1-N fusion protein was self-made; an anti-PD-1 antibody was purchased from Bioxcell; pan-02 pancreatic cancer cells were purchased from National Experimental Cell Resource Center; an aluminum hydroxide adjuvant was purchased from Corda. PE/DAZZLIE594-CD3 antibody (17A2 clone, rat IgG2b, κ), BV421-CD4 antibody (GK1.5 clone, rat IgG2b, κ), APC-FIRE75-CD8a (53-6.7 clone, rat IgG2a, κ), APC-CD335 (29A1.4 clone, rat IgG2a, κ), PE-CD19 (6D5 clone, rat IgG2a, κ), PE-CY7-F4/80 (BM8 clone, rat IgG2a, κ), FITC CD45 (30-F11 clone, rat IgG2b, κ), and BV711-CD11c (N418 clone, Armenian hamster IgG) were purchased from Biolegend; eFluor 506-L/D was purchased from Invitrogen; BB700CD11b (M1/70 clone, rat IgG2b, κ) was purchased from BD; a mouse tumor dissociation kit was purchased from Miltenyi.

Experimental animals: C57BL/6J mice were purchased from Beijing Huafukang Biotechnology Co., Ltd.

### 2. Methods

### 2.1 Immunization of mice

Mice were weighed and randomly divided into groups of 10. pan-02 pancreatic cancer cells were diluted with PBS and inoculated in the right axilla of the C57BL/J mice according to 3 × 10⁶ cells/0.1 mL per mouse, with a total amount of 100 µL/mouse, for modeling. After the tumor diameter reached 5 mm (10 days), the mice were divided into 5 groups, namely an aluminum hydroxide control group (100 µL, twice weekly, intramuscular injection), an MNRvax low-dose group (0.2 mg/kg, 100 µL, twice weekly, intramuscular injection), an MNRvax medium-dose group (2 mg/kg, 100 µL, twice weekly, intramuscular injection), an MNRvax high-dose group (8 mg/kg, 100 µL, twice weekly, subcutaneous injection), and an immune checkpoint inhibitor anti-PD-1 antibody group (10 mg/kg, 100 µL, twice weekly, intraperitoneal injection).

### 2.2 Mouse sample separation

### 2.2.1 Mouse tumor sample separation

A mixed enzyme solution of enzymes D, R, and A from Miltenyi was added to a tube, and tumor blocks with a diameter of 3 mm were added. The tube was mounted into a sleeve of the gentleMACS tissue processor, and the program 37C_m-TDK1 was run using the gentleMACS Octo tissue processor with a heating module. The sample was resuspended and filtered using a 70-µm cell strainer, and the cell suspension was collected in a 50-mL centrifuge tube. The strainer was rinsed with 20 mL of RPMI 1640. The cell suspension was centrifuged at 1500 rpm for 5 min, and the supernatant was discarded. 20 mL of PBS was added to resuspend the cells. The resulting mixture was well mixed by vortex and centrifuged at 1500 rpm for 5 min. The supernatant was discarded. The cells were resuspended in an appropriate volume of PBS. The resulting mixture was well mixed by vortex. 10 µL of the cell suspension was counted on a cell counter, and then the remaining cell suspension was centrifuged at 1500 rpm for 5 min. The supernatant was discarded. According to the counting results, an appropriate volume of PBS was added to resuspend the cells. The resulting mixture was well mixed by vortex, and the cell concentration was adjusted to 2 × 10⁷ cells/mL for later use.

### 2.2.2 Mouse spleen/lymph node cell dissociation

The collected spleen/lymph node was placed in a six-well plate containing 3 mL of a RPMI 1640 medium. The spleen was pressed with a syringe and then broken down into a single cell suspension. The cell strainer was placed on the top of a 15-mL conical tube, and the cell suspension in the six-well plate was passed through the strainer to remove cell pellets and debris. The strainer was rinsed with 5 mL of RPMI 1640. The cell suspension was centrifuged at 1500 rpm for 5 min, and the supernatant was discarded. 2 mL of a red blood cell lysis buffer was added to resuspend the cells. The resulting mixture was well mixed by vortex and centrifuged at 1500 rpm for 5 min. The supernatant was discarded. 10 mL of RPMI 1640 was added to resuspend the cells. The resulting mixture was well mixed by vortex and centrifuged at 1500 rpm for 5 min. The supernatant was discarded. An appropriate volume of a FACS buffer was added to resuspend the cells. The resulting mixture was counted and then centrifuged at 1500 rpm for 5 min. The supernatant was discarded. According to the counting results, an appropriate volume of the FACS buffer was added to resuspend the cells, and the cell concentration was adjusted to 2 × 10⁷ cells/mL for later use.

### 2.3 Mouse tumor/blood sample staining

Tumor cells, spleen cells, lymph node cells, and blood cells were separated at the end of the experiments for flow cytometry analysis of immune cell subsets of CD3, CD4, CD8, CD11, CD19, CD45, and CD335. CD45+ represents leukocytes; CD45+CD19+ represents B cells; CD45+CD19-CD3+ represents T cells; CD45+CD19-CD3+CD8+ represents cytotoxic T cells; CD45+CD19-CD3+CD4+ represents helper T cells; CD45+CD19-CD3-CD11b+F4/80+ represents macrophages; CD45+CD19-CD3-CD11c+ represents stellate cells; CD45+CD19-CD3-CD335+ represents natural killer cells.

The resuspended tumor/spleen/lymph node/blood cells were well mixed by vortex, and an FcR blocking reagent was added. The resulting mixture was incubated at 4 °C for 10 min in the dark. All antibodies (including live/dead dyes) were prepared into an antibody-mixed solution according to the recommended amount of the antibodies and were well mixed. An appropriate volume of the antibody-mixed solution was added to the corresponding flow cytometry tube, and an FMO tube, a blank tube, and a single positive tube were prepared according to requirements at the same time. The antibodies and the cells were well mixed by gentle vortex, and the resulting mixtures were incubated at 4 °C for 30 min in the dark. 2 mL of the red blood cell lysis buffer was added to each flow cytometry tube. The resulting mixtures were well mixed by vortex and incubated at room temperature for 10 min in the dark. After completion of the incubation, the mixtures were centrifuged at 1500 rpm for 5 min, and the supernatants were discarded. 2 mL of the FACS buffer was added to each flow cytometry tube. The resulting mixtures were well mixed by vortex and centrifuged at 1500 rpm for 5 min. The supernatants were discarded. The steps were repeated once. 50 µL of the FACS buffer was added to resuspend the cells. The resulting mixtures were well mixed by vortex, and loaded on the machine.

### 5.4 Data analysis

All data produced by the flow cytometer have been analyzed using the Kaluza software. In order to compare immune cell subsets of different treatment groups, we first validated the homogeneity of variance hypothesis among all groups using the Bartlett test. When the p value of the Bartlett test was not less than 0.05, one-way analysis of variance was used to test whether the mean values of all groups were equal. If the p value of the one-way analysis of variance was less than 0.05, we performed pairwise comparisons among all groups using the Tukey HSD test, or performed pairwise comparisons between each treatment group and the control group using the Dunnett's t test. When the p value of the Bartlett test was less than 0.05, the Kruskal Wallis test was used to test whether the medians of all groups were equal. If the p value of the Kruskal Wallis test was less than 0.05, we performed pairwise comparisons among all groups and pairwise comparisons between each treatment group and the control group using the Conover test, and performed the corresponding p value corrections according to the number of groups of the multiple tests. All statistical analysis and graphing were performed in the R language environment. All tests were two-tailed unless otherwise specified. A p value less than 0.05 was considered statistically significant.

### 3. Results

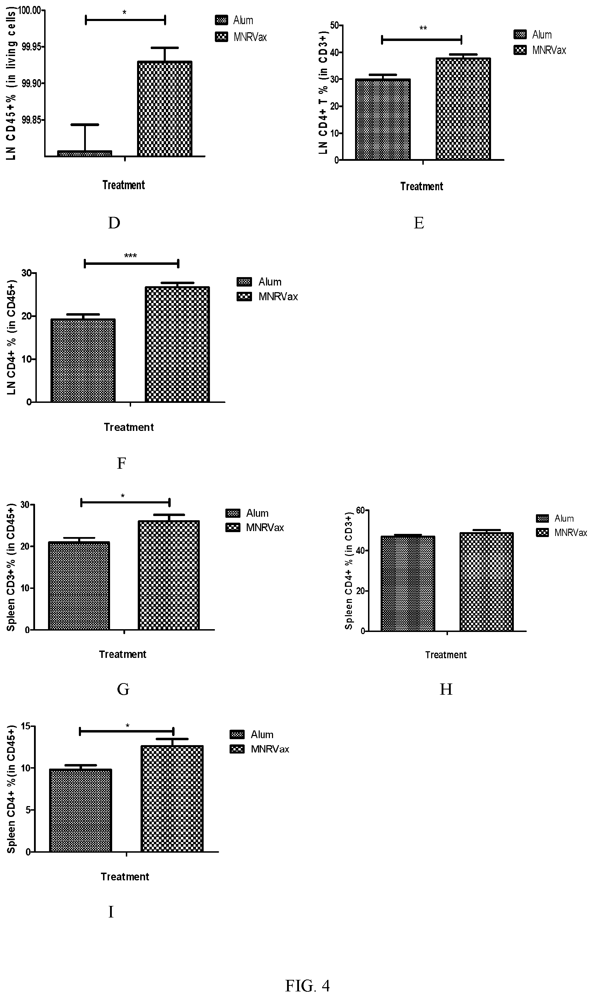

35 days after administration of the cancer vaccine, the percentage of leukocytes in the tumor was increased from 27.06±4.61 to 34.44±5.61 (p = 0.005) (FIG. 4A). In the tumor tissue, the proportion of CD8+ T cells in the total T cells was increased from 25.99±5.84% in the control group to 32.75±9.74% (p < 0.01) (FIG. 4B), and the proportion of CD8+ T cells in the total leukocytes was increased from 2.24±0.58% to 4.33±2.95% (p < 0.05) (FIG. 4C). 35 days after administration of the cancer vaccine, the percentage of leukocytes in the lymph node was increased from 99.81±0.12 to 99.93±0.06 (p = 0.011) (FIG. 4D). In the lymph node, the proportion of CD4+ T cells in the total T cells was increased from 29.85±5.96% in the control group to 37.75±4.71% (p < 0.01) (FIG. 4E), and the proportion of CD4+ T cells in the total leukocytes was increased from 19.22±3.73% to 26.70±3.3% (p < 0.001) (FIG. 4F). 35 days after administration of the cancer vaccine, the percentage of T cells in the spleen was increased from 20.91±3.55 to 26.03±4.93 (p = 0.017) (FIG. 4G). In the spleen, the proportion of CD4+ T cells in the total T cells was increased from 47.00±2.96% in the control group to 48.64±4.88% (p > 0.05) (FIG. 4H), and the proportion of CD4+ T cells in the total leukocytes was increased from 9.80±1.69% to 12.63±2.66% (p < 0.05) (FIG. 4I).

### 4. Conclusion

After inoculation of the MNRVax vaccine, CD8+ T lymphocytes in pancreatic cancer are increased significantly and directly kill cancer cells; CD8+ T lymphocytes in the lymph node are increased significantly, and total T cells in the spleen are increased significantly, suggesting that the inoculation of the MNRVax vaccine can mobilize immune cells into tumors.

The embodiments of the present disclosure have been described above. However, the present disclosure is not limited to the embodiments described above. Any modification, equivalent replacement, improvement, and the like made without departing from the spirit and principle of the present disclosure shall fall within the protection scope of the present disclosure.

The present invention can be summarized with the following items as follows:
1. Use of a fusion protein in the manufacture of a medicament for preventing and/or treating a tumor, characterized in that the tumor is a MUC1-positive tumor, an adenocarcinoma expressing MUC1, or a hematological tumor expressing MUC1; preferably, the tumor is pancreatic cancer.
2. Use of a fusion protein in the manufacture of a medicament for preventing and/or treating pancreatic cancer.
3. The use according to any one of items 1-2, characterized in that wherein the fusion protein comprises protein MBP gene and/or protein MUC1-N gene, preferably maltose-binding protein MBP gene and/or mucin MUC1-N gene.
4. The use according to any one of items 1-3, characterized in that wherein the fusion protein is formed by connecting the maltose-binding protein MBP gene and the mucin MUC1-N gene in tandem.
5. The use according to item3 or 4, characterized in that wherein the nucleotide sequence of the MUC1-N gene is set forth in SEQ ID NO.1, and the nucleotide sequence of the MBP gene is set forth in SEQ ID NO.2;
   preferably, the amino acid sequence of the fusion protein is set forth in SEQ ID NO.3.
6. Use of a fusion protein in a healthcare product, a cosmetic product, food, or a food additive, characterized in that wherein the fusion protein comprises protein MBP gene or protein and/or MUC1-N gene or protein, preferably maltose-binding protein MBP gene or protein and/or mucin MUC1-N gene or protein;
   preferably, the healthcare product, the cosmetic product, the food, or the food additive can be used for inhibiting a tumor;
   further preferably, the tumor is a MUC1-positive tumor, an adenocarcinoma expressing MUC1, or a hematological tumor expressing MUC1; more preferably, the tumor is pancreatic cancer.
7. The use according to item 6, characterized in that wherein the fusion protein is formed by connecting the binding protein MBP gene and the mucin MUC1-N gene in tandem;
   preferably, wherein the nucleotide sequence of the MUC1-N gene is set forth in SEQ ID NO.1, and preferably, the MBP gene is set forth in SEQ ID NO.2;
   preferably, the amino acid sequence of the fusion protein is set forth in SEQ ID NO.3.
8. Use of MBP gene or protein and/or MUC1-N gene in the manufacture of a medicament for preventing and/or treating a tumor, characterized in that the nucleotide sequence of the MUC1-N gene is set forth in SEQ ID NO.1, and the MBP gene is set forth in SEQ ID NO.2;
   preferably, the cancer comprises all cancers expressing MUC1, including adenocarcinomas expressing MUC1 or hematological tumors expressing MUC1, more preferably pancreatic cancer.
9. Use of protein MBP gene and/or protein MUC1-N gene in the manufacture of a medicament for preventing and/or treating pancreatic cancer, characterized in that the nucleotide sequence of the MUC1-N gene is set forth in SEQ ID NO.1, and the MBP gene is set forth in SEQ ID NO.2.
10. A pharmaceutical composition for preventing and/or treating a tumor, comprising a fusion protein formed by connecting maltose-binding protein (MBP) gene and mucin MUC1-N gene in tandem, an aluminum hydroxide adjuvant, and a CpG adjuvant, the amino acid sequence of the fusion protein is set forth in SEQ ID NO.3, and preferably, the CpG adjuvant is partially or fully thio-modified.
11. Use of the composition according to item 10 in the manufacture of a medicament for preventing and/or treating pancreatic cancer.

## Claims

1. A fusion protein, wherein the amino acid sequence of the fusion protein is set forth in SEQ ID NO: 3.

2. A polynucleotide comprising a maltose-binding protein MBP gene and a mucin MUC1-N gene connected in tandem, wherein the nucleotide sequence of the MUC1-N gene is set forth in SEQ ID NO: 1, and the nucleotide sequence of the MBP gene is set forth in SEQ ID NO: 2, and the polynucleotide encodes a fusion protein having the amino acid sequence set forth in SEQ ID NO: 3.

3. A recombinant host cell comprising the polynucleotide according to claim 2, wherein the recombinant host cell is an Escherichia coli cell.

4. A method for producing a fusion protein, comprising culturing the recombinant host cell according to claim 3 under conditions permitting expression of the polynucleotide and recovering the expressed fusion protein, wherein the amino acid sequence of the fusion protein is set forth in SEQ ID NO: 3.

5. A pharmaceutical composition comprising the fusion protein according to claim 1.

6. The pharmaceutical composition according to claim 5, further comprising an aluminum hydroxide adjuvant and a CpG adjuvant.

7. The pharmaceutical composition according to claim 6, wherein the CpG adjuvant is partially or fully thio-modified.

8. The pharmaceutical composition according to claim 6, wherein the CpG adjuvant is CpG1826 or fully thio-modified CpG1826.
